(19) Europäisches Patentamt / European Patent Office / Office européen des brevets

(11) **EP 2 832 346 B1**

(12) **EUROPEAN PATENT SPECIFICATION**

(45) Date of publication and mention
of the grant of the patent:
**10.10.2018 Bulletin 2018/41**

(21) Application number: **13769727.2**

(22) Date of filing: **06.02.2013**

(51) Int Cl.:
*A61K 8/67* *(2006.01)*          *A61K 8/06* *(2006.01)*
*A61K 8/37* *(2006.01)*          *A61K 8/55* *(2006.01)*
*A61K 47/14* *(2017.01)*         *A61K 47/22* *(2006.01)*
*A61K 47/24* *(2006.01)*         *A61Q 19/00* *(2006.01)*
*A61K 8/39* *(2006.01)*          *A61K 8/00* *(2006.01)*
*A61K 8/02* *(2006.01)*          *A61K 8/04* *(2006.01)*
*A61K 9/00* *(2006.01)*          *A23L 33/15* *(2016.01)*

(86) International application number:
**PCT/JP2013/052763**

(87) International publication number:
**WO 2013/145870 (03.10.2013 Gazette 2013/40)**

(54) **AQUEOUS DISPERSED COMPOSITION**

DISPERGIERTE WÄSSRIGE ZUSAMMENSETZUNG

COMPOSITION DISPERSÉE AQUEUSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2012   JP 2012080124
08.06.2012   JP 2012130956
25.01.2013   JP 2013012533**

(43) Date of publication of application:
**04.02.2015 Bulletin 2015/06**

(73) Proprietor: **FUJIFILM Corporation
Tokyo 106-8620 (JP)**

(72) Inventor: **MURAGUCHI, Taichi
Ashigarakami-gun
Kanagawa 258-8577 (JP)**

(74) Representative: **Klunker IP
Patentanwälte PartG mbB
Destouchesstraße 68
80796 München (DE)**

(56) References cited:
WO-A1-98/53836          DE-A1-102008 035 834
GB-A- 2 453 157         JP-A- H04 270 218
JP-A- H08 127 526       JP-A- 2002 226 402
JP-A- 2002 338 499      JP-A- 2004 161 733
JP-A- 2008 154 577      JP-A- 2009 256 268
KR-B1- 100 614 816      US-A- 5 376 646
US-A- 6 066 328         US-A1- 2003 149 100
US-A1- 2005 214 240     US-A1- 2007 041 922
US-A1- 2011 305 735

- SANGSTER J: "OCTANOL-WATER PARTITION
COEFFICLENTS OF SIMPLE ORGANIC
COMPOUNDS", JOURNAL OF PHYSICAL AND
CHEMICAL REFERENCE DATA, AMERICAN
CHEMICAL SOCIETY, NEW YORK, NY, US, vol.
18, no. 3, 1 January 1989 (1989-01-01), pages
1111-1229, XP000667323, ISSN: 0047-2689
- "Partition coefficient", wikipedia , XP002744551,
Retrieved from the Internet:
URL:https://en.wikipedia.org/wiki/Partitio
n_coefficient [retrieved on 2015-09-14]

## Description

Technical Field

The present invention relates to an aqueous dispersion composition and uses thereof.

Background Art

Naturally occurring type vitamin E compounds are fat-soluble vitamins and have moderate antioxidant ability, and thus are widely known as antioxidants for drugs, foods, feeds, and food additives for nutrition or the like. Recently, naturally occurring type vitamin E compounds are widely used also in supplements, cosmetics, and the like.

Meanwhile, dispersion techniques are techniques which are widely applied for the purpose of improvement in disposition in the living body or enhancement in skin permeability, or, from a more familiar point of view, for the purpose of, for example, improvement in appearance or feeling of use of beverage or cosmetics in general due to the transparency provided thereby. In order to effectively use naturally occurring type vitamin E compounds, which have the antioxidant ability as well as physiological functionality, various studies have been made on dispersions containing naturally occurring type vitamin E compounds (European Patent Application Publication No. 2133066 and Japanese Patent Application Laid-Open (JP-A) No. 2001-151664).

Particularly, since naturally occurring type vitamin E compounds are not stable, studies are being made to develop a dispersion having favorable stability over time of dispersed particles and favorable appearance that is free of clouding or the like, through a decrease in the particle sizes of dispersed particles, addition of components that contribute to stability, or the like.

For example, JP-A No. 2001-151664 describes that mixing of a retinoid, a vitamin E compound, and an amino alcohol results in improvement of the chemical stability of the retinoid as well as improvement of the chemical stability of vitamin E compound.

Further, JP-A No. 2004-168702 discloses a fine emulsion composition in which ethylene oxide-added hydrogenated castor oil and glycerin are used together with a vitamin A and a vitamin E compound.

Moreover, JP-A No. 2004-285078 discloses a skin care composition which has a pH within a specific range and contains a chelating agent.

DE 10 2008 035834 A1 discloses a method for preparing a liposome complex. Further disclosed are smaller liposomes which are included in larger liposomes. The smaller liposomes have a particle diameter of 50 to 200 nm while the larger liposomes have a particle diameter of 250 to 600 nm.

US 5,376,646 A teaches topical preparations containing a salt of a cholanic acid, a lipid, and optionally, one or more other pharmaceutically or cosmetically active substances. These preparations are used in pharmaceutical and/or cosmetic applications.

KR 100614816 B1 teaches a cosmetic composition containing nanocapsules. The composition may include caprylic acid and triglycerides of caprylic and capric acids which act as a barrier to help the softener (emollient), and water binding to the skin.

US 6,066,328 A relates to a cosmetic or dermatological composition comprising an emulsion of oil-in-water type formed of oily globules which are each provided with a lamellar liquid crystal coating and are dispersed in an aqueous phase. Each oily globule containing at least one lipophilic compound which is cosmetically or dermatologically active is individually coated with a mono lamellar or oligolamellar layer obtained from at least one lipophilic surface-active agent, from at least one hydrophilic surface-active agent and from at least one ionic amphiphilic lipid imparting to the emulsion a pH ranging from 5.5 to 7.5, the coated oily globules having a mean diameter of less than 500 nanometers.

JP 2004-161733 A relates to an emulsion-based cosmetic that stably contains vitamin A and/or a fatty acid ester thereof, which has good feeling, high spreadability and no greasiness, shows skin roughening amelioration properties and high storage stability.

JP 2009-256268 A teaches aqueous stabilized compositions for stabilizing a retinoid of a physiologically active component which is effective for the efficacy of the improvement of wrinkles, firmness, rough skin and the like.

SUMMARY OF INVENTION

Technical Problem

However, dispersions are generally in a semi-stable state, and stability thereof cannot be maintained over time, and it is known that an increase in the particle size during storage eventually leads to a higher tendency towards the separation of the water phase and the oil phase from each other. Therefore, depending on the type of container, there are cases in which dispersion stability is significantly deteriorated.

Further, decreased particle sizes of dispersed particles in the case of fine dispersed particles cause an exponential increase in the surface area serving as the reaction field, and may give rise to a problem in term of decomposition of naturally occurring type vitamin E compounds.

An object of the invention is to provide an aqueous dispersion composition in which decomposition of a naturally occurring type vitamin E compound, having poor stability, is suppressed to ensure stability of the compound itself, and which has excellent stability of dispersed particles, and further provide an external skin preparation and a functional food, each of which includes the aqueous dispersion composition.

Solution to Problem

The invention includes the following.

[1] An aqueous dispersion composition comprising dispersed particles that include: a naturally occurring type Vitamin E compound comprising at least one selected from the group consisting of tocopherols and tocotrienols; retinyl palmitate; an oil agent having a calculated octanol-water partition coefficient (cLogPow) of from 0 to 12, wherein cLogPow is a value determined by a quantitative structure-activity correlation algorithm, or a value determined by a fragment method, and wherein the oil agent comprises tri(caprylic acid/capric acid)glyceride; and a phospholipid, wherein a particle diameter of the dispersed particles is from 20 nm to 90 nm, wherein the particle diameter is measured using a dynamic light scattering method.

[2] The aqueous dispersion composition according to [1], wherein a content of the retinyl palmitate is from 0.001 times to 200 times the content of the naturally occurring type vitamin E compound, in terms of mass ratio.

[3] The aqueous dispersion composition according to [1] or [2], wherein the phospholipid is lecithin.

[4] The aqueous dispersion composition according to any one of [1] to [3], wherein the cLogPow of the oil agent is from 2 to 12.

[5] The aqueous dispersion composition according to [1], wherein the content of the naturally occurring type Vitamin E compound is from 0,0001% by mass to 10% by mass with respect to the total mass of the aqueous dispersion composition.

[6] The aqueous dispersion composition according to [1], further comprising a surfactant.

[7] The aqueous dispersion composition according to [1], wherein the content of the phospholipid is from 0.001% by mass to 10% by mass with respect to the total mass of the aqueous dispersion composition.

[8] An external skin preparation including the aqueous dispersion composition according to any one of [1] to [7].

[9] A functional food including the aqueous dispersion composition according to any one of [1] to [7].

Advantageous Effects of Invention

According to the invention, an aqueous dispersion composition can be provided in which decomposition of a naturally occurring type vitamin E compound, having poor stability, is suppressed to ensure stability of the compound itself and which has excellent stability of dispersed particles. Further, an external skin preparation and functional food, each of which includes the aqueous dispersion composition, can also be provided.

BRIEF DESCRIPTION OF DRAWINGS

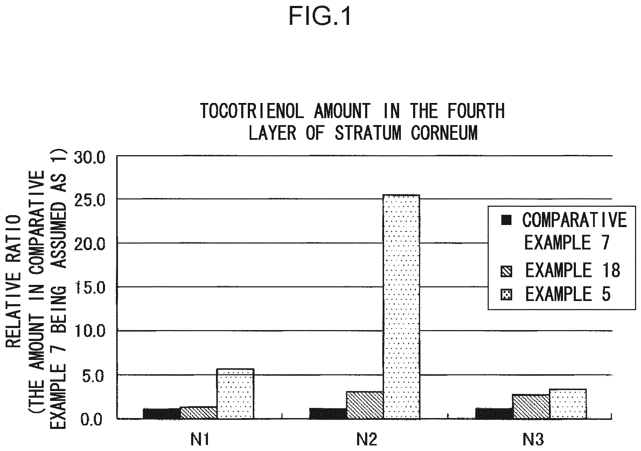

Fig. 1 is a graph in which the stratum corneum fourth layer tocotrienol amounts in Example 5 and Example 18 according to the invention and in Comparative Example 7 are compared.

EMBODIMENTS FOR CARRYING OUT INVENTION

The aqueous dispersion composition according to the invention is an aqueous dispersion composition including dispersed particles that include: a naturally occurring type vitamin E compound comprising at least one selected from the group consisting of tocopherols and tocotrienols; retinyl palmitate; an oil agent having an calculated octanol-water partition coefficient (cLogPow) of from 0 to 12 , wherein cLogPow is a value determined by a quantitative structure-activity correlation algorithm, or a value determined by a fragment method, and wherein the oil agent comprises tri(caprylic acid/capric acid)glyceride; and a phospholipid.

In the aqueous dispersion composition according to the invention, a naturally occurring type vitamin E compound comprising at least one selected from the group consisting of tocopherols and tocotrienols and retinyl palmitate, together with an oil agent having an calculated octanol-water partition coefficient (cLogPow) of from 0 to 12 and comprising tri(caprylic acid/capric acid)glyceride, and a phospholipid, constitute dispersed particles, which are dispersed in a water phase. Since the oil agent has properties such that the cLogPow thereof is from 0 to 12, the oil agent is presumed to have a tendency to confine the naturally occurring type vitamin E compound inside the dispersed particles, and thus the stability of the naturally occurring type vitamin E compound is maintained. Further, there is a tendency for the presence of retinyl palmitate to effectively suppress enlargement of the particle sizes of the dispersed particles. As a result, an aqueous dispersion composition in which decomposition of a naturally occurring type vitamin E compound, having poor stability, is suppressed to ensure stability of the compound itself, and which has excellent stability of dispersed particles, is obtained. However, the invention is not limited by the specific theory discussed above.

In the present specification, the scope of the term "process" includes not only an independent process, but also a process that cannot be clearly distinguished from another process, as long as the predetermined purpose of the process of interest is achieved.

In the specification, the expression "(from)...to..." denotes a range including numerical values before and after "to" as the minimum value and the maximum value, respectively.

In the specification, in a case in which the amount of a component that may be included in the composition is indicated, when there are plural substances corresponding to the component in the composition, the indicated amount means the total amount of the plural substances present in the composition, unless specifically stated otherwise.

In the invention, the term "water phase" is used as a word that contrasts with the term "oil phase", regardless of the kind of solvent.

Hereinafter, the invention is described.

[Aqueous Dispersion Composition]

The aqueous dispersion composition according to the invention is an aqueous dispersion composition including dispersed particles that include a naturally occurring type vitamin E compound comprising at least one selected from the group consisting of tocopherols and tocotrienols, retinyl palmitate, an oil agent having a calculated partition coefficient (cLogPow) in water and octanol of from 0 to 12 and comprising tri(caprylic acid/capric acid)glyceride, and a phospholipid.

The aqueous dispersion composition is a composition having an oil-in-water type emulsion configuration in which dispersed particles that include a naturally occurring type vitamin E compound, retinyl palmitate, an oil agent, a phospholipid and, if necessary, other components are dispersed as an oil phase in a water phase. The components capable of constituting the dispersed particles, including the naturally occurring type vitamin E compound and retinyl palmitate, are also referred to as simply "oil phase components". Further, the components capable of constituting the water phase are hereinafter also referred to as simply "water phase components".

(Naturally occurring type Vitamin E Compound)

The term "naturally occurring type vitamin E compound" means a vitamin E that can be found in nature or a derivative thereof. According to the present invention, the naturally occurring type vitamin E compound is at least one selected from the group consisting of tocopherols and tocotrienols.

Tocopherols include dl-$\alpha$-tocopherol, dl-$\beta$-tocopherol, dl-$\gamma$-tocopherol, and dl-$\delta$-tocopherol. These tocopherols may be used singly, or in mixture of two or more thereof. In particular, as the tocopherol, a mixture of the above four types of tocopherols (mixed tocopherol) is preferable in terms of availability of raw materials, high purity at the time of purification, and safety to the living body, and the like.

Tocotrienols are tocopherol analogs that are contained in wheat, barley, rice bran, palm oil, and the like, each have three double bonds in the side chain moiety of a tocopherol, and has excellent antioxidant performance. Tocotrienols include $\alpha$-tocotrienol, $\beta$-tocotrienol, $\gamma$-tocotrienol, and $\delta$-tocotrienol. These substances may be used singly, or in mixture of two or more thereof. Among them, the tocotrienol is preferably a mixture of the above four types of tocotrienols, in consideration of availability of raw materials, high purity at the time of purification, safety with respect to the living body, and the like.

There is no particular limitation as to the content of naturally occurring type vitamin E compound in the aqueous dispersion composition. However, from the viewpoint of more reliable exertion of the predetermined function of naturally occurring type vitamin E compounds, the content of naturally occurring type vitamin E compound is preferably from 0.0001% by mass to 10% by mass, and more preferably from 0.001% by mass to 1% by mass, with respect to the total mass of the aqueous dispersion composition. Further, the content of naturally occurring type vitamin E compound is preferably from 0.1% by mass to 50% by mass, and more preferably from 1% by mass to 20% by mass, with respect to the total mass of the oil phase of the aqueous dispersion composition.

(Retinyl Palmitate)

Retinyl palmitate is an ester of palmitic acid and retinol, and may also be referred to as "vitamin A palmitate". In the aqueous dispersion composition according to the invention, the stability over time of the dispersed particles is favorably maintained by retinyl palmitate.

The content of retinyl palmitate in the aqueous dispersion composition is preferably from 0.001 times to 200 times the content of naturally occurring type vitamin E compound in terms of mass ratio, more preferably from 0.01 times to 30 times the content of naturally occurring type vitamin E compound in terms of mass ratio, and still more preferably from 0.1 times to 10 times the content of naturally occurring type vitamin E compound in terms of mass ratio, from the viewpoints of the stability over time of the aqueous dispersion composition and the stability of the naturally occurring type vitamin E compound.

(Oil agent)

The oil agent is a hydrophobic oil agent having a calculated octanol-water partition coefficient (cLogPow) of from 0 to 12. However, the naturally occurring type vitamin E compound and retinyl palmitate are not included in the scope of the oil agent.

The term "oil agent" as used in the invention means a substance which is liquid at 25°C and phase-separates from water.

The calculated octanol-water partition coefficient (cLogPow; hereinafter, LogP value) is a value that is generally expressed in common logarithm. The LogP value is one of the values representing the properties of chemical substances, and is a constant value that is independent of addition amount. The LogP value is a value expressed by a common logarithm obtained from the concentration in the water phase and the concentration in the octanol phase in a system of a mixed liquid of water and n-octanol in an equilibrium state in which the water phase and the octanol phase contact each other. There is a tendency for a greater LogP value to indicate a relative increase in hydrophobicity, and there is also a tendency for a smaller LogP value to indicate a relative increase in hydrophilicity.

The LogP value in the invention is a value calculated according to the calculation method known in the related art. Further, as the LogP value to be used may be a value determined through calculation using commercially available software or the like in which quantitative structure-activity correlation algorithm is used. Depending on the kind of oil agent, the fragment method may be adopted. In the fragment method, a partition coefficient of a molecule is calculated based on the sum of the respective partition coefficients of the partial structures of the molecule.

Examples of commercially available software capable of calculating a LogP value by calculation include CHEMDRAW PRO 12.0. The LogP value may alternatively be a value obtained by utilizing http://www.vcclab.org/lab/alogps/start.html.

The oil agent has a LogP value of from 0 to 12. There is a tendency that a LogP value of the oil agent of more than 12 results in migration of components, such as naturally occurring type vitamin E compounds, toward the outside (i.e., toward the water phase side) in the interior of the dispersed particles, and thus the stability of naturally occurring type vitamin E compounds is deteriorated. Further, since the oil agent is an oil agent having hydrophobicity, substances having hydrophilicity such as alcohols, namely, substances having a LogP value of less than zero, are not included in the scope of the oil agent.

The LogP value of the oil agent is preferably from 2 to 12, more preferably from 4 to 12, still more preferably from 6 to 12, and further more preferably from 6 to 10, from the viewpoint of the stability of dispersed particles.

The oil agent comprises tri(caprylic acid/capric acid)glyceride (9.25). The oil agent may further include diisopropyl adipate (2.04), tetradecanal (4.67), isopropyl myristate (4.42), myristic acid (4.94), propylene glycol dicaprylate (5.47), 1-methylundecane (5.51), stearic acid (6.61), triethylhexanoin (7.05), isooctyl palmitate (8.86), and tocopherol acetate (10.61). Note that, the numbers written inside the parentheses in the above examples represent LogP values.

There is no particular limitation on the content of oil agent. From the viewpoint of more reliable exertion of the predetermined function of the naturally occurring type vitamin E compound, the content of oil agent is preferably 0.0001 times to 10 times the content of naturally occurring type vitamin E compound in terms of mass ratio, and more preferably from 0.001 times to 2 times the content of the naturally occurring type vitamin E compound in terms of mass ratio.

The aqueous dispersion composition may include, besides the oil agent having a LogP value of from 0 to 12, an oil agent having a LogP of more than 12 (hereinafter, referred to as "additional oil agent"), to the extent of not impairing the effects of the invention.

However, phospholipids and surfactants, which are described below, are not included in the scope of the oil agent and the additional oil agent.

The kind of the additional oil agent is not particularly limited, as long as the additional oil agent is a component that does not dissolve in an aqueous medium but dissolves in an oil-based medium, and the additional oil agent is a substance that can generally be included, as an oily component, in an aqueous dispersion composition. An additional oil agent having desired physical properties or functionality can be selected, as appropriate, and used. Examples of oil agents and additional oil agents that can be included in the aqueous dispersion composition include: ceramide compounds, including ceramides and ceramide analogs, for example, naturally occurring type ceramides and sugar-modified ceramides such as sphingoglycolipid; oils and fats such as olive oil, Camellia Japonica seed oil, Macadamia Ternifolia seed oil, castor oil, and coconut oil; hydrocarbons such as liquid paraffin, paraffin, Vaseline, ceresin, microcrystalline wax, and squalane; waxes such as carnauba wax, candelilla wax, jojoba oil, beeswax, and lanolin; esters such as 2-octyldodecyl myristate, cetyl 2-ethylhexanoate, and diisostearyl malate; fatty acids such as palmitic acid and isostearic acid; higher alcohols such as cetyl alcohol, stearyl alcohol, isostearyl alcohol, or 2-octyldodecanol; silicone oils such as methyl polysiloxane or methylphenyl polysiloxane; glycerin fatty acid esters; fat-soluble vitamins such as vitamin As and vitamin Ds; oil soluble dyes such as carotenoids such as lycopene and astaxanthin; polymers; and oil soluble proteins. Further examples include various plant-derived oils and animal-derived oils, which are mixtures of substances selected from the above substances. However, retinyl palmitate and naturally occurring type vitamin E compounds are not included in the oil agent and the additional oil agent.

(Surfactant)

The aqueous dispersion composition may contain a surfactant as a dispersant component. As the surfactant in the invention, surfactant emulsifying agents that dissolve in an aqueous medium (hydrophilic surfactants) are preferable due to their capacity to greatly decrease the interfacial tension between the oil phase and the water phase in the dispersion composition, and to resultantly realize fine particle diameters.

As the surfactant, from the viewpoint of dispersion stability of the aqueous dispersion composition, surfactants having HLB of 8 or more are preferable, substances having HLB of 10 or more are more preferable, and substances having HLB of 12 or more are particularly preferable. The upper limit of the HLB value is not particularly limited. The upper limit of the HLB value is generally 20 or less, and preferably 18 or less.

Here, the HLB represents a hydrophilicity-hydrophobicity balance, and is generally used in the field of surfactants. Generally used calculation formulas, for example, the Kawakami's formula, may be used therefor. In the invention, the Kawakami's formula as shown below is adopted.

$$HLB = 7 + 11.7 \log(Mw/Mo)$$

Here, Mw represents the molecular weight of hydrophilic group(s), and Mo represents the molecular weight of hydrophobic group(s).

Further, numerical values of HLB described in catalogues and the like may be used.

As can be understood from the above formula, surfactants having desired HLB values can be obtained by utilizing the additive property of HLB.

Examples of the surfactant include cationic surfactants, anionic surfactants, amphoteric surfactants, and nonionic surfactants. There is no particular limitation on the surfactant, and nonionic surfactants are preferable. Examples of nonionic surfactants include organic acid monoglycerides, polyglycerin fatty acid esters, polyglycerin condensed ricinoleic acid esters, sorbitan fatty acid esters, sucrose fatty acid esters, and polyoxyethylene sorbitan fatty acid esters. Polyglycerin fatty acid esters, sorbitan fatty acid esters, sucrose fatty acid esters, and polyoxyethylene sorbitan fatty acid esters are more preferable. It is not essential that the surfactant be highly purified by distillation or the like. The surfactant may be a reaction mixture.

A polyglycerin fatty acid ester that can be used in the invention is an ester of a polyglycerin having an average polymerization degree of 2 or more, preferably from 6 to 15, and more preferably from 8 to 10, and a fatty acid having from 8 to 18 carbon atoms, for example, caprylic acid, capric acid, lauric acid, myristic acid, palmitic acid, stearic acid, oleic acid, or linolic acid. Preferable examples of the polyglycerin fatty acid ester include hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate, and decaglycerin monolaurate.

Among them, decaglycerin monooleate (HLB = 12), decaglycerin monostearate (HLB = 12), decaglycerin monopalmitate (HLB = 13), decaglycerin monomyristate (HLB = 14), decaglycerin monolaurate (HLB = 16), and the like are more preferable.

These polyglycerin fatty acid esters may be used singly, or in mixture.

(Phospholipid)

The aqueous dispersion composition includes a phospholipid from the viewpoint of, for example, stability of dispersed particles. The phospholipid may be an ester formed from a fatty acid, an alcohol, phosphoric acid, and a nitrogen-containing compound, among complex lipids. Phospholipids form a group of compounds including phosphoric acid esters and fatty acid esters. Phospholipids include glycerophospholipids and sphingophospholipids.

Examples of glycerophospholipids include phosphatidic acid, bisphosphatidic acid, lecithin (phosphatidylcholine), phosphatidylethanolamine, phosphatidylmethylethanolamine, phosphatidylserine, phosphatidylinositol, phosphatidylglycerin, and diphosphatidylglycerin (cardiolipin). Further examples include materials that are derived from plants such as soybeans, corn, peanuts, rapeseeds, wheat, and barley and that contain a glycerophospholipid as described above, materials that are derived from animals such as egg yolk and cows and that contain a glycerophospholipid as described above, and various lecithins that are derived from, for example, microorganisms such as *Escherichia coli* and that contain a glycerophospholipid as described above.

Examples of sphingophospholipids include sphingomyelin. Further, in the invention, an enzyme-decomposed glycerophospholipid may be used as the glycerophospholipid.

For example, lysolecithin (enzyme-decomposed lecithin) obtained by enzymatic decomposition of lecithin is a compound formed by removal of either of the fatty acid moiety (acyl group) bonded at the 1-position or the fatty acid moiety (acryl group) bonded at the 2-position from a glycerophospholipid. A decrease in the number of fatty acid groups to 1 improves the hydophilicity of lecithin, and enhances the emulsifiability or dispersibility of lecithin in water.

Lysolecithin can be obtained by hydrolyzing lecithin using an acid or an alkali catalyst, and can also be obtained by hydrolyzing lecithin using phospholipase A1 or A2.

Examples of compound names of lyso compounds, a typical example of which is lysolecithin, include lysophosphatidic acid, lysophosphatidylglycerin, lysophosphatidylinositol, lysophosphatidylethanolamine, lysophosphatidylmethylethanolamine, lysophosphatidylcholine (lysolecithin), and lysophosphatidylserine. Further, hydrogenated or hydroxylated products obtained from glycerophospholipids typified by the lecithin are also usable in the invention.

Among the above compounds, lecithin and lysolecithin, which are glycerophospholipids, are preferable as the phospholipid contained in the aqueous dispersion composition, from the viewpoint of emulsion stability, and lecithin is more preferable. The lecithin as used in the invention may be a substance having a purity of 60% by mass or higher, and is preferably a substance having a lecithin purity of 80% by mass or higher, which is generally referred to as "high purity lecithin", and more preferably a substance having a lecithin purity of 90% by mass or higher. The lecithin purity (% by mass) is determined by subtracting the weight of matter that does not dissolve in toluene and the weight of matter that dissolves in acetone, utilizing the property that lecithin easily dissolves in toluene but does not dissolve in acetone. High purity lecithin has high lipophilicity as compared with lysolecithin; it is therefore conceivable that the high lipophilicity heightens the compatibility between lecithin and oily components, and improves emulsion stability. The phospholipid for use in the invention may be used singly or in the form of a mixture of plural kinds thereof.

The content of phospholipid is preferably from 0.01% by mass to 30% by mass, more preferably from 0.01% by mass to 20% by mass, and still more preferably from 0.1% by mass to 20% by mass, with respect to the total mass of oil agents (in a case in which an additional oil agent is present, with respect to the total mass of additional oil agents and oil agents), from the viewpoint of dispersion stability of the aqueous dispersion composition.

Further, the content of phospholipid is preferably from 0.001% by mass to 10% by mass of the whole composition. There is a tendency that a phospholipid content within such a range provides favorable emulsion stability of the aqueous dispersion composition.

[Water Phase]

The water phase that constitutes the aqueous dispersion composition together with the dispersed particles may be formed of an aqueous medium, especially water. The water phase may further include, if necessary, a water-soluble component, for example, a polyhydric alcohol or a surfactant.

[Method of Producing Aqueous Dispersion Composition]

A method of producing the aqueous dispersion composition according to the invention is not particularly limited. For example, a preferred production method includes the processes of a) obtaining a water phase by optionally dissolving one or more water phase components in an aqueous medium (water or the like), b) obtaining an oil phase by mixing oil phase components (a naturally occurring type vitamin E compound, retinyl palmitate, an oil agent, a phospholipid, and the like) for dissolution, and c) mixing the water phase and the oil phase under stirring and performing emulsification/dispersion, thereby obtaining an aqueous dispersion composition.

The components contained in the oil phase and the water phase in the production method are as described in the above explanation of the constituent components of the aqueous dispersion composition according to the invention, and preferable examples thereof and preferable amounts thereof are also the same as those of the constituent components of the aqueous dispersion composition described above.

The ratio (by mass) of the oil phase to the water phase in the emulsification/dispersion is not particularly limited, and the oil phase/water phase ratio (% by mass) is preferably from 0.1/99.9 to 50/50, more preferably from 0.5/99.5 to 30/70, and still more preferably from 1/99 to 20/80.

An oil phase/water phase ratio of 0.1/99.9 or higher is preferable since there is a tendency that such a ratio avoids occurrence of problems associated with practical application of the dispersion composition by preventing the amounts of active ingredients from becoming too small. Further, an oil phase/water phase ratio of 50/50 or lower is preferable since there is a tendency that such a ratio avoids deterioration of the emulsion stability of the dispersion composition by preventing the surfactant concentration from becoming too low.

The emulsification/dispersion may be conducted by carrying out a one-step emulsification operation. However, it is preferable to carry out an emulsification operation having two or more steps, from the viewpoint of obtaining uniform and fine dispersed particles.

Specifically, it is particularly preferable to use two or more types of emulsification apparatuses in combination in a manner such that, for example, emulsification through a high-pressure homogenizer, an ultrasonic dispersing machine, or the like is performed in addition to a one-step emulsification operation in which emulsification is performed using an ordinary emulsification apparatus utilizing shear action (for example, a stirrer, an impeller stirrer, a homomixer, or a continuous-flow shearing apparatus). Use of a high-pressure homogenizer enables the dispersion to include liquid droplets in the form of more uniform fine particles. Such emulsification/dispersion operations may be performed plural times for the purpose of obtaining liquid droplets having more uniform particle diameters.

The temperature condition in the emulsification/dispersion in the invention is not particularly limited, and the temperature is preferably from 10°C to 100°C from the viewpoint of stability of functional oily components. A preferable range may be selected, as appropriate, according to, for example, the melting points of the oil phase components to be employed.

In a case in which a high-pressure homogenizer is used in the invention, processing is preferably carried out at a pressure of preferably 50 MPa or higher, more preferably from 50 MPa to 280 MPa, and still more preferably from 100 MPa to 280 MPa.

From the viewpoint of maintaining the particle sizes of dispersed particles, the emulsion liquid, which is a composition obtained by the emulsification/dispersion, is preferably cooled by a cooling device within 30 seconds, preferably within 3 seconds, after the emulsion liquid has passed through the chamber.

[Particle Diameter of Dispersed Particle in Aqueous Dispersion Composition]

In the water phase of the aqueous dispersion composition, dispersed particles are dispersed as the oil phase. The particle diameter of the dispersed particles is from 20 nm to 90 nm, from the viewpoints of, for example, stability and favorable appearance of the aqueous dispersion composition, and the systemic absorbability or percutaneous absorbability of the components that constitute the aqueous dispersion composition.

The particle diameter of the dispersed particles in the aqueous dispersion composition may be measured using a commercially available particle size distribution meter or the like. Various methods for measuring particle size distributions of dispersions are known. In the measurement of the particle diameter in the invention, a dynamic light scattering method is used.

In the invention, the particle diameter is a value measured using a FIBER-OPTICS PARTICLE ANALYZER FPAR-1000 (Otsuka Electronics Co., Ltd.), and, specifically, a value measured in the manner described below is adopted.

The method of measuring the particle diameter is as follows. The measurement is conducted using a quartz cell after dilution with pure water to adjust the concentration of oil phase components to 1% by mass is performed. The median diameter (d=50) as measured with parameter settings including a refractive index of a sample of 1.600, a refractive index of the dispersion medium of 1.333 (pure water), and a viscosity of the dispersion medium set to the viscosity of pure water is obtained and employed as the particle diameter.

[Use]

The aqueous dispersion composition according to the invention is a composition which stably includes a naturally occurring type vitamin E compound and has excellent stability over time; for example, enlargement of dispersed particles is suppressed over a long-term. Accordingly, the aqueous dispersion composition can preferably be applied to uses in which favorable and continuous exertion of physiological functions of naturally occurring type vitamin E compound is desired. Specific examples of such uses include an external skin preparation, which is desired to locally exert an effect when topically administered to the skin, and a functional food, which is desired to exert an effect in a wide area through systemic absorption when orally administered.

[External Skin Preparation]

An external skin preparation according to the invention includes the aqueous dispersion composition according to the invention. This configuration enables the external skin preparation to stably include a stable naturally occurring type vitamin E compound and have excellent stability over time.

The external skin preparation according to the invention may be applied to uses such as cosmetic materials or percutaneous pharmaceuticals.

Examples of the external skin preparation according to the invention include, but are not limited to, skin cosmetic materials (for example, skin lotion, essence, milky lotion, and cream), lipsticks, sun block cosmetic materials, makeup cosmetic materials, face packs, masks, shampoo cosmetics, fragrance cosmetics, liquid body cleansing materials, UV care cosmetics, deodorant cosmetics, oral care cosmetics, and cosmetics for scalp and hair.

The addition amount of the aqueous dispersion composition according to the invention in the external skin preparation varies depending on the type and purpose of products and cannot unconditionally be specified; the aqueous dispersion composition for use may be added in an amount such that the content of the aqueous dispersion composition is in a range of from 0.01% by mass to 10% by mass, preferably from 0.05% by mass to 5% by mass, with respect to the product. When the addition amount is 0.01% by mass or more, exertion of the desired effect can be sufficiently expected, and when the addition amount is 10% by mass or less, an appropriate effect can generally be efficiently exerted.

The external skin preparation according to the invention may include, besides the aqueous dispersion composition according to the invention, ingredients that can be added to ordinary external skin preparations, as desired, to the extent at which the effects of the invention are not impaired.

Examples of such other components include, but are not limited to, ultraviolet absorbents, ultraviolet scattering agents, waxes, hydrocarbon oils, fatty acid esters, silicone oils, polyhydric alcohols, water-soluble polymers, higher alcohols, and higher fatty acids.

The external skin preparation may include, besides the aqueous dispersion composition according to the invention, one or more of: vitamins such as vitamin A and vitamin A derivatives, vitamin C and vitamin C derivatives, vitamins D, vitamins E, and pantothenic acid; cyclic oligosaccharides such as α-cyclodextrin, β-cyclodextrin, and γ-cyclodextrin; extracts from plants such as scutellaria baicalensis root, curcumin, prunus mume, eriobotrya japonica leaf, brown algae, yeast, mugwort, aloe, gardenia, sponge gourd, and pepperwort; α-hydroxy acids such as glycolic acid; and the like.

[Functional Food]

A functional food according to the invention includes the aqueous dispersion composition according to the invention. This configuration enables the functional food to include a stable naturally occurring type vitamin E compound and have excellent stability over time. Here, the term "functional food" as used in the invention means a food that can be expected to exert a desired physiological function in the living body, based on the function unique to the component contained therein.

To the functional food, ingredients that can be used in food applications may be added, as necessary.

Milk proteins, soybean proteins, egg albumin protein, and the like, or white egg oligopeptides, soybean hydrolysis products, and mixtures of single amino acids, which are degradation products of the above substances, may be used as ingredients that can be used in food applications. Sugars, fats, trace elements, vitamins, emulsifiers, fragrances, and the like may be incorporated. For example, in order to improve the nutritional balance or the flavor at the time of ingestion, nutritional additives, such as amino acids, vitamins, and minerals, sweeteners, spices, dyes, and the like may be incorporated.

Preferable examples of the form of the functional food according to the invention include, but are not limited to, general foods such as energy drinks, nutritive analeptics, luxury drinks, and frozen sweets, as well as dietary supplements in the form of tablets, granules, capsules, or retort-packed form.

The addition amount of the aqueous dispersion composition according to the invention to be contained in the functional food according to the invention varies depending on the type and purpose of products and cannot unconditionally be specified; the aqueous dispersion composition for use may be added in an amount such that the content of the aqueous dispersion composition is in a range of from 0.01% by mass to 10% by mass, more preferably from 0.01% by mass to 5% by mass, with respect to the product. When the addition amount is 0.01% by mass or more, exertion of the desired effect can be sufficiently expected, and when the addition amount is 10% by mass or less, an appropriate effect can generally be efficiently exerted.

EXAMPLES

Hereinafter, the present invention will be described in detail with reference to Examples. Note that, the "%" and "parts" are based on mass unless specified otherwise.

[Reference Example 1]

The following ingredients were dissolved under heating at 70°C for one hour, to obtain a water phase composition.

| | |
|---|---|
| Decaglyceryl monooleate (HLB =12) | 94.0 g |
| Glycerin | 446.0 g |
| Pure water | 275.0g |

Further, the following ingredients were dissolved under heating at 70°C for one hour, to obtain an oil phase composition.

| | |
|---|---|
| Retinyl palmitate | 1.5 g |
| Tocopherol | 61 g |
| Tocopherol acetate | 103.5 g |
| Lecithin (derived from soybean) | 19.0 g |

In the above, the decaglyceryl monooleate employed was "NIKKOL DECAGLYN 1-OV" (Nikko Chemicals Co., Ltd.), the retinyl palmitate employed was "RIKEN A PALMITATE 1000 (E)" (Riken Vitamin Co., Ltd.), the tocopherol employed was a mixed tocopherol ("RIKEN E OIL 800", Riken Vitamin Co., Ltd.), and the lecithin employed was "SLP WHITE" (Tsuji Oil Mill Co., Ltd.).

The above water phase composition was stirred (10,000 rpm) using a homogenizer (Model HP93, manufactured by SMT Co.) while the temperature of the water phase composition was maintained at 70°C, and the above oil phase composition was added to the water phase composition, to obtain a pre-dispersed material.

Subsequently, the pre-dispersed material thus obtained was cooled to about 60°C, and was subjected to high-pressure emulsification at a pressure of 245 MPa using an ULTIMIZER HJP-25005 (manufactured by Sugino Machine Limited). Then, the resultant was filtered through a microfilter having an average pore size of 1 μm to obtain an aqueous dispersion composition (Sample 1) containing a naturally occurring type vitamin E compound.

[Examples 2, 5, 7 to 16, Reference Examples 3, 4, 6 and 17, and Comparative Examples 1 to 6]

Sample 2 to Sample 23 were obtained in the same manner as in Reference Example 1, except that the kind of naturally occurring type vitamin E compound, the kind or content of oil agent, the content of retinyl palmitate, and the content of lecithin were changed as indicated in Table 2. In Table 2, "%" means a content percentage relative to the total mass of the aqueous dispersion composition.

In Table 2, the tocotrienol employed was "TOCOLIT 92" (Eisai food & Chemical Co., Ltd.), the fatty acid (C8, C10) triglyceride employed was "COCONAD MT" (Kao Corporation), the isopropyl myristate employed was "IPM" (Nippon Fine Chemical Co., Ltd.), and the squalane employed was "SQUALANE" (Maruha Nichiro Foods, Inc.).

<Evaluation>

With regard to each of Sample 1 to Sample 23 obtained as described above, evaluation on the stability of the dispersion composition was performed as follows.

(1) Initial Particle Diameter

1.0 g of the sample immediately after preparation was added to 99.0 g of pure water, and the mixture was stirred for 10 minutes using a magnetic stirrer. The particle diameter of the obtained water-diluted dispersion composition liquid was obtained as a median diameter (d = 50) as measured by a FIBER-OPTICS PARTICLE ANALYZER FPAR-1000 (Otsuka Electronics Co., Ltd.) at 25°C with parameter settings including a refractive index of the sample of 1.600, a refractive index of the dispersion medium of 1.333 (pure water), and a viscosity of the dispersion medium set to the viscosity of pure water. The results are indicated in Table 2 below.

(2) Evaluation of Stability Over Time

Each of the obtained samples was allowed to stand in a thermostat chamber under accelerated conditions of 40°C, 50°C for one month (28 days). After the passage of this period, the sample was left to stand until the sample temperature reached room temperature, and the particle diameter was measured in the same manner as that described in evaluation (1) above. Based on the ratio of change of particle diameter between just after preparation and after the passage of the period, evaluation was made as follows. The results are indicated in Table 2.

S: The ratio of change from the initial particle diameter is 150% or less.
A: The ratio of change from the initial particle diameter is from more than 150% to 175%.
B: The ratio of change from the initial particle diameter is from more than 175% to 200%.
C: The ratio of change from the initial particle diameter is more than 200%.

(3) Evaluation on Stability of Naturally Occurring Type Vitamin E Compound

For the evaluation on the stability of the naturally occurring type vitamin E compound, the samples were individually diluted with a 50 mM citrate buffer (pH 7.0) to a final concentration of 0.2%, thereby providing evaluation samples. Each of the evaluation samples was left to stand at 40°C for 28 days, and the content of naturally occurring type vitamin E compound was measured under the conditions described below according to a gradient analysis method using a high speed liquid chromatography (HPLC). Further, the content of naturally occurring type vitamin E compound in the respective samples just after the test was also measured under the same measurement conditions. Evaluation was performed as follows, based on the residual ratio of naturally occurring type vitamin E compound determined from the content of naturally occurring type vitamin E compound at the initiation of the test and the residual amount of naturally occurring type vitamin E compound after the test. The results are indicated in Table 2.
(HPLC Conditions)

| Column: | C8 5 μm 4.6 mm × 150 mm |
|---|---|
| Flow rate: | 1 mL/min |
| Column temperature: | 40°C |
| Detection wavelength: | 297 nm |

Developing solution

A: methanol/ water = 75/25 (volume ratio; including 0.1% by volume of acetic acid and 0.1% by volume of triethanolamine)
B: methanol (including 0.1% by volume of acetic acid and 0.1% by volume of triethanolamine)

(Gradient Condition)

The gradient condition was set as follows.

TABLE 1

| Time (min) | B. Pump. (%) |
|---|---|
| 0.1 | 20 |
| 20 | 100 |
| 30 | 100 |
| 30.1 | 20 |
| 45 | Stop |

(Evaluation)

A: The residual ratio of naturally occurring type vitamin E compound is 80% or higher.
B: The residual ratio of naturally occurring type vitamin E compound is from 70% to lower than 80%.
C: The residual ratio of the naturally occurring type vitamin E compound is lower than 70%.

(4) Comprehensive Evaluation

From the results of evaluations (2) and (3) above, an aqueous dispersion composition having excellent stability is ranked "E", an aqueous dispersion composition having favorable stability is ranked "G", an aqueous dispersion composition having inferior stability is ranked "NG". The results are shown in Table 2. In Table 2, Samples 1, 3, 6, 8 and 23 are Reference Examples.

TABLE 2

| Sample No. | Naturally Occurring Type Vitamin E | | Oil agent | | | Retinyl Palmitate Content (2) | (2)/(1) ratio (%) | Lecithin Content | Evaluation | | | | Note |
|---|---|---|---|---|---|---|---|---|---|---|---|---|---|
| | Kind | Content (1) | Kind | Log P | Content | | | | Initial Particle Diameter (nm) | Particle Size Stability Over Time | Residual Ratio after Passage of Time | Comprehensive Evaluation | |
| 1 | Tocopherol | 6.1% | Tocopherol acetate | 10.61 | 10.35% | 0.15% | 2.5 | 1.90% | 55.9 | S | A | E | Reference Example 1 |
| 2 | Tocopherol | 6.1% | Fatty acid (C8,C10) triglyceride | 9.25 | 10.35% | 0.15% | 2.5 | 1.90% | 60.1 | S | A | E | Example 2 |
| 3 | Tocopherol | 6.1% | Isopropyl myristate | 4.42 | 10.35% | 0.15% | 2.5 | 1.90% | 59.6 | A | A | G | Reference Example 3 |
| 4 | Tocopherol | 6.1% | Tocopherol | 9.98 | 10.35% | 0.15% | 2.5 | 1.90% | 56.1 | S | C | NG | Comparative Example 1 |
| 5 | Tocopherol | 6.1% | Squalane | 12.49 | 10.35% | 0.15% | 2.5 | 1.90% | 53.8 | S | C | NG | Comparative Example 2 |
| 6 | Tocotrieno l | 6.1% | Tocopherol acetate | 10.61 | 10.35% | 0.15% | 2.5 | 1.90% | 59.1 | S | A | E | Reference Example 4 |
| 7 | Tocotrieno l | 6.1% | Fatty acid (C8,C10) triglyceride | 9.25 | 10.35% | 0.15% | 2.5 | 1.90% | 57.4 | S | A | E | Example 5 |
| 8 | Tocotrieno l | 6.1% | Isopropyl myristate | 4.42 | 10.35% | 0.15% | 2.5 | 1.90% | 59.6 | A | A | G | Reference Example 6 |
| 9 | Tocotrieno l | 6.1% | Tocotrienol | 9.98 | 10.35% | 0.15% | 2.5 | 1.90% | 64.7 | S | C | NG | Comparative Example 3 |
| 10 | Tocotrieno l | 6.1% | Squalane | 12.49 | 10.35% | 0.15% | 2.5 | 1.90% | 60.1 | S | C | NG | Comparative Example 4 |
| 11 | Tocopherol | 6.1% | Fatty acid (C8,C10) triglyceride | 9.25 | 10.50% | 0.00% | 0.0 | 1.90% | 48.7 | C | B | NG | Comparative Example 5 |

(continued)

| Sample No. | Naturally Occurring Type Vitamin E | | Oil agent | | | Retinyl Palmitate Content (2) | (2)/(1) ratio (%) | Lecithin Content | Evaluation | | | | Note |
|---|---|---|---|---|---|---|---|---|---|---|---|---|---|
| | Kind | Content (1) | Kind | Log P | Content | | | | Initial Particle Diameter (nm) | Particle Size Stability Over Time | Residual Ratio after Passage of Time | Comprehensive Evaluation | |
| 12 | Tocopherol | 6.1% | Fatty acid (C8,C10) triglyceride | 9.25 | 10.49% | 0.02% | 0.2 | 1.90% | 47.5 | S | A | E | Example 7 |

| Kind | Content (1) | Kind | LogP | Content | Initial Particle Diameter (nm) | Particle Size Stability Over Time | Residual Ratio after Passage of Time | Comprehensive Evaluation | | | | | |
|---|---|---|---|---|---|---|---|---|---|---|---|---|---|
| 13 | Tocopherol | 6.1% | Fatty acid (C8,C10) triglyceride | 9.25 | 9.00% | 1.50% | 25 | 1.90% | 58.1 | S | A | E | Example 8 |
| 14 | Tocopherol | 6.1% | Fatty acid (C8,C10) triglyceride | 9.25 | 0.01% | 10.49% | 172 | 1.90% | 78.5 | A | B | G | Example 9 |
| 15 | Tocotrienol | 6.1% | Fatty acid (C8,C10) triglyceride | 9.25 | 10.50% | 0.00% | 0.0 | 1.90% | 57.4 | C | B | NG | Comparative Example 6 |
| 16 | Tocotrienol | 6.1% | Fatty acid (C8,C10) triglyceride | 9.25 | 10.49% | 0.02% | 0.2 | 1.90% | 55.6 | S | A | E | Example 10 |
| 17 | Tocotrienol | 6.1% | Fatty acid (C8,C10) triglyceride | 9.25 | 9.00% | 1.50% | 25 | 1.90% | 61.9 | S | A | E | Example 11 |
| 18 | Tocotrienol | 6.1% | Fatty acid (C8,C10) triglyceride | 9.25 | 0.01% | 10.49% | 172 | 1.90% | 89.6 | A | B | G | Example 12 |

EP 2 832 346 B1

| Kind | Content (1) | Kind | LogP | Content | Initial Particle Diameter (nm) | Particle Size Stability Over Time | Residual Ratio after Passage of Time | Comprehensive Evaluation | | | | | |
|---|---|---|---|---|---|---|---|---|---|---|---|---|---|
| 19 | Tocopherol | 6.1% | Fatty acid (C8,C10) triglyceride | 9.25 | 10.35% | 0.15% | 2.5 | 2.85% | 72.8 | A | A | E | Example 13 |
| 20 | Tocopherol | 6.1% | Fatty acid (C8,C10) triglyceride | 9.25 | 10.35% | 0.15% | 2.5 | 3.80% | 86.5 | B | A | G | Example 14 |
| 21 | Tocotrienol | 6.1% | Fatty acid (C8,C10) triglyceride | 9.25 | 10.35% | 0.15% | 2.5 | 2.85% | 74.3 | A | A | E | Example 15 |
| 22 | Tocotrienol | 6.1% | Fatty acid (C8,C10) triglyceride | 9.25 | 10.35% | 0.15% | 2.5 | 3.80% | 80.2 | B | A | G | Example 16 |
| 23 | Tocopherol | 6.1% | Triethylhexanoin | 7.05 | 10.35% | 0.00% | 2.5 | 1.90% | 54.2 | S | A | E | Reference Example 17 |

As shown in Table 2, each of Sample 1 to Sample 3, Sample 6 to Sample 8, Sample 12 to Sample 14, and Sample 16 to Sample 23 according to the Examples according to the invention and Reference Examples 1, 3, 4, 6, and 17 was an aqueous dispersion composition having favorable stability over time which exhibited little decomposition of naturally occurring type vitamin E compound and a low degree of enlargement of dispersed particles.

In contrast, Sample 4 (Comparative Example 1) and Sample 9 (Comparative Example 3), which did not further include an oil agent having a LogP value of 12 or less in addition to the naturally occurring type vitamin E compound, and Sample 5 (Comparative Example 2) and Sample 10 (Comparative Example 4), which did not include an oil agent having a LogP value of 12 or less, exhibited a residual ratio of naturally occurring type vitamin E compound of lower than 70%, and could not stably include the naturally occurring type vitamin E compound.

Further, Sample 11 (Comparative Example 5) and Sample 15 (Comparative Example 6), which did not contain retinyl palmitate, could not maintain the ratio of change of the particle diameter of the dispersed particles from the initial particle diameter under the conditions of 40°C, 50°C for one month (28 days) within the range of 200% or less, and the stability of the dispersed particles was deteriorated.

[Example 18 and Comparative Example 7]

(Stratum Corneum Permeation Test)

Sample 24 (Example 18) was prepared in the same manner as the preparation of Sample 7 (Example 5), except that the pre-dispersed material was subjected to ultrasonic dispersion instead of the high-pressure emulsification. Sample 25 (Comparative Example 7) was prepared by mixing tocotrienol (final concentration: 6.1% by mass) and water (final concentration: 93.9% by mass) and then subjecting the resultant mixture to ultrasonic dispersion. Using the samples of Example 18 and Comparative Example 7 obtained, comparison was made with respect to the permeability of the aqueous dispersion compositions into stratum corneum. Further, the stratum corneum permeation test was also performed on Example 5 described above, and comparison with Comparative Example 7 was made, similar to the comparison of Example 18 and Comparative Example 7.

Note that, the initial particle diameters of Example 18 and Comparative Example 7 were measured in the same manner as that in the measurement in Reference Example 1 described above.

As a result, the initial particle diameter of the sample of Example 18 was 160 nm, and the initial particle diameter of the sample of Comparative Example 7 was from 1 $\mu$m to 10 $\mu$m. Further, as indicated in Table 2, the initial particle diameter of Sample 7 of Example 5 was 57.4 nm.

(Evaluation Method)

Example 5, Example 18, and Comparative Example 7 were individually impregnated into FINN CHAMBER (registered trademark) patches (Taisho Pharmaceutical Co., Ltd.) in an amount of 15 $\mu$L each. The upper arms of three adults (two males and one female) in their 30's were closely patched with these patches. After 4 hours, the patches were removed, and the parts to which the patches had been attached were washed well. After the washing, in order to analyze the tocotrienol amount in the fourth layer of stratum corneum, air-drying was performed for 30 minutes, and a procedure of separating the stratum corneum layer by layer using tape stripping was repeated 4 times, thereby obtaining the fourth layer.

The obtained tape with the fourth layer was immersed in 500 $\mu$L of ethanol, and ultrasonic dispersion (US-103, SND Co., Ltd.) was carried out for 30 minutes, whereby tocotrienol was extracted. Subsequently, the amount of tocotrienol that had permeated into the stratum corneum was analyzed using HPLC. The HPLC conditions applied were the same conditions as the conditions described above. Evaluation was performed based on the relative value (ratio by mass), assuming that the amount of tocotrienol in Comparative Example 7 was 1.

The results are indicated in Fig. 1 and Table 3.

TABLE 3

|  | N1 | N2 | N3 |
|---|---|---|---|
| Comparative Example 7 | 1 | 1 | 1 |
| Example 18 | 1.2 | 3 | 2.6 |
| Example 5 | 5.6 | 25.4 | 3.1 |

As shown in Fig. 1 and Table 3, in all of the subjects, Example 18 exhibited improved permeability of tocotrienol, as compared with Comparative Example 7.

Further, as shown in Example 5, a minute particle diameter achieved by high-pressure dispersion remarkably improved the permeability of tocotrienol in all of the subjects. Particularly, in subject N2, the permeability of Example 5 was about 25 times higher than Comparative Example 7.

Accordingly, it was revealed that, by using the aqueous dispersion composition according to the invention, not only the naturally occurring type vitamin E compound was stably contained, but also the permeability thereof into the skin was significantly improved. Although the above description employed Example 5 and Example 18 as examples, it goes without saying that similar effects can be obtained in other embodiments of the invention.

[Reference Example 19]

Beverage was prepared with the following composition and production method, using the aqueous dispersion composition (Sample 1) obtained in Reference Example 1.

<Composition>

| | |
|---|---|
| (1) Aqueous dispersion composition of Reference Example 1 | 20 g |
| (2) Fructose glucose syrup | 120 g |
| (3) Vitamin C (L-ascorbic acid) | 10 g |
| (4) Citric acid | 10 g |
| (5) Orange perfume | 3 g |
| (6) Water | 837 g |
| Total | 1000 g |

The above ingredients (2) to (6) were mixed for dissolution. Thereafter, ingredient (1) was added thereto, followed by further mixing, as a result of which a liquid for drink was prepared. This liquid was filled into a bottle, and pasteurized under heating at 85°C for 10 minutes. The resulting liquid was cooled to room temperature, to obtain beverage.

The beverage thus obtained had excellent transparency, and even when the beverage was allowed to stand, change such as clouding was not observed.

[Reference Example 20]

Next, a skin lotion was prepared with the following composition and production method, using the aqueous dispersion composition (Sample 1) obtained in Reference Example 1.

<Composition>

| | |
|---|---|
| (1) 1,3-Butanediol | 60 g |
| (2) Glycerin | 40 g |
| (3) Oleyl alcohol | 1 g |
| (4) Polyoxyethylene (20) sorbitan monolauric acid ester | 5 g |
| (5) Polyoxyethylene (15) lauryl alcohol ether | 5 g |
| (6) Ethanol | 100 g |
| (7) Methylparaben | 2 g |
| (8) Sodium L-Ascorbate | 10 g |
| (9) Aqueous dispersion composition of Reference Example 1 | 1 g |
| (10) Purified water | 776 g |
| Total | 1000 g |

Ingredient (1) was added to and dissolved in ingredient (10), to obtain a water phase. Subsequently, ingredients (2) to (5), (7), and (8) were dissolved in ingredient (6) at 45°C, and then mixed with the water phase with stirring. Further, ingredient (9) was added thereto, followed by stirring and mixing, to obtain a skin lotion.

The skin lotion thus obtained had excellent transparency, and, even when the skin lotion was left to stand and stored at 50°C for 3 months, clouding was not recognized.

[Formulation Example]

Hereinafter, examples of formulations for a cosmetic material in which the aqueous dispersion composition of Example 2 is used as an ingredient for adding tocopherol and in which the aqueous dispersion composition of Example 5 is used as an ingredient for adding tocotrienol are described. In the formulations described below, in cases in which decaglyceryl monooleate, glycerin, retinyl palmitate, lecithin, caprylic/capric triglycerides , or water is present as an ingredient to be added, the amounts of these ingredients in the aqueous dispersion compositions of Example 2 and Example 5 are not included in the indicated contents of the these ingredients.

The formulation examples described below were prepared according to ordinary methods.

Also in the formulations described below, the aqueous dispersion compositions of Example 2 and Example 5 exert an effect in terms of favorable stability over time, such as little decomposition of naturally occurring type vitamin E compound and low degree of enlargement of dispersed particles including naturally occurring type vitamin E compound.

(1) Cleansing Oil-1

| | |
|---|---|
| Mineral oil | Remainder (about 70% by mass) |
| Isopropyl palmitate | 10% by mass |
| PEG-20 glyceryl triisostearate | 10% by mass |
| Water | 5% by mass |
| 1,3-Butylene glycol (hereinafter referred to as "BG") | 1% by mass |
| Stearyl glycyrrhetinate | 0.2% by mass |
| Sorbeth-30 tetraoleate | 0.2% by mass |
| Aqueous dispersion composition of Example 5 | 0.2% by mass |
| Aqueous dispersion composition of Example 2 | 0.2% by mass |
| Retinyl palmitate | 0.2% by mass |
| Scutellaria baicalensis root extract (Ichimaru Pharcos Co., Ltd., the same shall apply hereinafter) | 0.1% by mass |
| Cyclodextrin (Ichimaru Pharcos Co., Ltd., the same shall apply hereinafter) | 0.001% by mass |
| Turmeric root extract (Maruzen Pharmaceuticals Co., Ltd., the same shall apply hereinafter) | 0.001% by mass |
| Orange oil | 0.001% by mass |
| Glycerin | 0.001% by mass |
| Caprylic/capric triglycerides | 0.001% by mass |
| Corn oil | 0.001% by mass |
| Lecithin | 0.001% by mass |
| Polyglyceryl-10 oleate | 0.001% by mass |
| Fragrance | 0.001% by mass |
| Phenoxyethanol | 0.1% by mass |
| Ethylparaben | 0.1% by mass |

(2) Cleansing Oil-2

| | |
|---|---|
| Ethylhexyl palmitate | Remainder (about 50% by mass) |
| Polyglyceryl-10 trilaurate | 20% by mass |
| Caprylic/capric triglycerides | 20% by mass |
| Stearyl glycyrrhetinate | 0.2% by mass |

(continued)

| | |
|---|---|
| Aqueous dispersion composition of Example 5 | 0.4% by mass |
| Aqueous dispersion composition of Example 2 | 0.1% by mass |
| Retinyl palmitate | 0.1% by mass |
| Scutellaria baicalensis root extract | 0.005% by mass |
| Cyclodextrin | 0.0001% by mass |
| Turmeric root extract | 0.0001% by mass |
| Orange oil | 0.0001% by mass |
| BG | 0.0001% by mass |
| Corn oil | 0.0001% by mass |
| Lecithin | 0.0001% by mass |
| Polyglyceryl-10 oleate | 0.0001% by mass |
| Glycerin | 0.0001% by mass |
| Water | 0.0001% by mass |
| Fragrance | 0.0001% by mass |
| Phenoxyethanol | 0.0001% by mass |

(3) Cleansing Gel

| | |
|---|---|
| Triethylhexanoin | Remainder (about 40% by mass) |
| Glycerin | 40% by mass |
| BG | 10% by mass |
| PEG-20 glyceryl triisostearate | 2% by mass |
| Water | 2% by mass |
| Stearyl glycyrrhetinate | 0.2% by mass |
| Aqueous dispersion composition of Example 5 | 0.2% by mass |
| Aqueous dispersion composition of Example 2 | 0.1% by mass |
| Retinyl palmitate | 0.1% by mass |
| Scutellaria baicalensis root extract | 0.05% by mass |
| Cyclodextrin | 0.00001% by mass |
| Turmeric root extract | 0.00001% by mass |
| Orange oil | 0.00001% by mass |
| Triethanolamine (TEA) | 0.00001% by mass |
| (Acrylates/ alkyl acrylate (C10-30)) crosspolymer | 0.00001% by mass |
| Corn oil | 0.00001% by mass |
| Caprylic/capric triglycerides | 0.00001% by mass |
| Lecithin | 0.00001% by mass |
| Polyglyceryl-10 oleate | 0.00001% by mass |
| Phenoxyethanol | 0.001% by mass |

(4) Cleansing Foam

| | |
|---|---|
| Water | Remainder (about 60% by mass) |
| Glycerin | 10% by mass |
| Sodium cocoyl glycinate | 10% by mass |
| Sodium lauroamphoacetate | 10% by mass |
| Citric acid | 2% by mass |
| Dipotassium glycyrrhizinate | 0.3% by mass |

(continued)

| | |
|---|---|
| BG | 0.1% by mass |
| Aqueous dispersion composition of Example 5 | 0.2% by mass |
| Aqueous dispersion composition of Example 2 | 0.08% by mass |
| Retinyl palmitate | 0.1% by mass |
| Scutellaria baicalensis root extract | 0.05% by mass |
| Cyclodextrin | 0.008% by mass |
| Turmeric root extract | 0.005% by mass |
| Glycolic acid | 0.005% by mass |
| Polyquaternium-39 | 0.005% by mass |
| Hydroxypropyl methylcellulose | 0.005% by mass |
| Corn oil | 0.005% by mass |
| Caprylic/capric triglycerides | 0.005% by mass |
| Lecithin | 0.005% by mass |
| Polyglyceryl-10 oleate | 0.005% by mass |
| Disodium EDTA | 0.000001% by mass |
| Fragrance | 0.005% by mass |
| Phenoxyethanol | 0.005% by mass |

(5) Essence

| | |
|---|---|
| Water | Remainder (about 60% by mass) |
| Dimethicone | 10% by mass |
| BG | 5% by mass |
| Ethanol | 5% by mass |
| Pentylene glycol | 5% by mass |
| Diphenylsiloxy phenyl trimethicone | 5% by mass |
| Polysorbate 60 | 1% by mass |
| Dipotassium glycyrrhizinate | 0.5% by mass |
| Aqueous dispersion composition of Example 5 | 1% by mass |
| Aqueous dispersion composition of Example 2 | 1% by mass |
| Retinyl palmitate | 0.2% by mass |
| Scutellaria baicalensis root extract | 0.01% by mass |
| Cyclodextrin | 0.008% by mass |
| Turmeric root extract | 0.008% by mass |
| Prunus mume fruit extract | 0.005% by mass |
| Eriobotrya japonica leaf extract | 0.005% by mass |
| Laminaria ochroleuca extract | 0.005% by mass |
| Saccharomyces ferment lysate filtrate | 0.005% by mass |
| Glycolic acid | 0.005% by mass |
| Glycerin | 0.005% by mass |
| (Acrylates/ alkyl acrylate (CI0-30)) crosspolymer | 0.005% by mass |
| Xanthan gum | 0.005% by mass |
| Corn oil | 0.005% by mass |
| Caprylic/capric triglycerides | 0.005% by mass |
| Lecithin | 0.005% by mass |
| Polyglyceryl-10 oleate | 0.005% by mass |
| Disodium EDTA | 0.005% by mass |
| Potassium hydroxide | 0.005% by mass |
| Phenoxyethanol | 0.1% by mass |

(6) Lotion-1

| Water | Remainder (about 80% by mass) |
|---|---|
| Dipropylene glycol (DPG) | 5% by mass |
| BG | 1% by mass |
| Glycerin | 1% by mass |
| PEG-32 | 0.5% by mass |
| Trehalose | 0.1% by mass |
| Dipotassium glycyrrhizinate | 0.05% by mass |
| Aqueous dispersion composition of Example 5 | 0.03% by mass |
| Aqueous dispersion composition of Example 2 | 0.02% by mass |
| Retinyl palmitate | 0.02% by mass |
| Scutellaria baicalensis root extract | 0.01% by mass |
| Cyclodextrin | 0.01% by mass |
| Turmeric root extract | 0.001% by mass |
| Prunus mume fruit extract | 0.001% by mass |
| Eriobotrya japonica leaf extract | 0.001% by mass |
| Laminaria ochroleuca extract | 0.001% by mass |
| Corn oil | 0.001% by mass |
| Caprylic/capric triglycerides | 0.001% by mass |
| Lecithin | 0.001% by mass |
| Polyglyceryl-10 oleate | 0.001% by mass |
| PEG-60 hydrogenated castor oil | 0.001% by mass |
| Sodium citrate | 0.001% by mass |
| Citric acid | 0.001% by mass |
| Phenoxyethanol | 0.001% by mass |
| Methylparaben | 0.001% by mass |

(7) Lotion-2

| Water | Remainder (about 70% by mass) |
|---|---|
| BG | 5% by mass |
| Glycerin | 5% by mass |
| Methyl gluceth-20 | 1% by mass |
| Pentylene glycol | 0.5% by mass |
| Xylitol | 0.1% by mass |
| Dipotassium glycyrrhizinate | 0.08% by mass |
| Aqueous dispersion composition of Example 5 | 0.05% by mass |
| Aqueous dispersion composition of Example 2 | 0.05% by mass |
| Retinyl palmitate | 0.005% by mass |
| Scutellaria baicalensis root extract | 0.005% by mass |
| Cyclodextrin | 0.00001% by mass |
| Turmeric root extract | 0.00001% by mass |
| Prunus mume fruit extract | 0.000005% by mass |
| Eriobotrya japonica leaf extract | 0.000005% by mass |
| Laminaria ochroleuca extract | 0.000005% by mass |
| Sodium hyaluronate | 0.000005% by mass |
| Hydroxyethyl cellulose | 0.000005% by mass |
| Corn oil | 0.000005% by mass |

(continued)

| | |
|---|---|
| Caprylic/capric triglycerides | 0.000005% by mass |
| Lecithin | 0.000005% by mass |
| Polyglyceryl-10 oleate | 0.000005% by mass |
| PEG-60 hydrogenated castor oil | 0.000005% by mass |
| Sodium citrate | 0.000005% by mass |
| Citric acid | 0.0005% by mass |
| Phenoxyethanol | 0.01% by mass |
| Methylparaben | 0.01% by mass |

(8) Lotion-3

| | |
|---|---|
| Water | Remainder (about 60% by mass) |
| Glycerin | 15% by mass |
| BG | 10% by mass |
| Methyl gluceth-20 | 10% by mass |
| PEG-32 | 2% by mass |
| Pentylene glycol | 1% by mass |
| Xylitol | 0.2% by mass |
| Dipotassium glycyrrhizinate | 0.2% by mass |
| Aqueous dispersion composition of Example 5 | 0.2% by mass |
| Aqueous dispersion composition of Example 2 | 0.1% by mass |
| Retinyl palmitate | 0.05% by mass |
| Scutellaria baicalensis root extract | 0.05% by mass |
| Cyclodextrin | 0.005% by mass |
| Turmeric root extract | 0.001% by mass |
| Prunus mume fruit extract | 0.001% by mass |
| Eriobotrya japonica leaf extract | 0.001% by mass |
| Laminaria ochroleuca extract | 0.001% by mass |
| Sodium hyaluronate | 0.001% by mass |
| Hydroxyethyl cellulose | 0.001% by mass |
| Betaine | 0.001% by mass |
| Corn oil | 0.001% by mass |
| Caprylic/capric triglycerides | 0.001% by mass |
| Lecithin | 0.001% by mass |
| Polyglyceryl-10 oleate | 0.001% by mass |
| PEG-60 hydrogenated castor oil | 0.001% by mass |
| Sodium citrate | 0.001% by mass |
| Citric acid | 0.001% by mass |
| Phenoxyethanol | 0.001% by mass |
| Methylparaben | 0.001% by mass |

(9) Milk-1

| | |
|---|---|
| Water | Remainder (about 70% by mass) |
| Glycerin | 5% by mass |
| BG | 3% by mass |
| Cyclopentasiloxane | 3% by mass |

(continued)

| | |
|---|---|
| Dipotassium glycyrrhizinate | 0.3% by mass |
| Squalane | 0.1% by mass |
| Aqueous dispersion composition of Example 5 | 0.2% by mass |
| Aqueous dispersion composition of Example 2 | 0.2% by mass |
| Retinyl palmitate | 0.2% by mass |
| Scutellaria baicalensis root extract | 0.0005% by mass |
| Cyclodextrin | 0.0001% by mass |
| Turmeric root extract | 0.0001% by mass |
| Prunus mume fruit extract | 0.0005% by mass |
| Eriobotrya japonica leaf extract | 0.0005% by mass |
| Laminaria ochroleuca extract | 0.0005% by mass |
| Arginine | 0.0005% by mass |
| Dimethicone | 0.0005% by mass |
| Maltitol | 0.0005% by mass |
| Glyceryl caprylate | 0.0005% by mass |
| Phytosteryl/octyldodecyl lauroyl glutamate | 0.0005% by mass |
| Xanthan gum | 0.0005% by mass |
| Corn oil | 0.0005% by mass |
| Caprylic/capric triglycerides | 0.0005% by mass |
| Lecithin | 0.0005% by mass |
| Polyglyceryl-10 oleate | 0.0005% by mass |
| PEG-400 | 0.0005% by mass |
| (Acrylates/ alkyl acrylate (CI0-30)) crosspolymer | 0.0005% by mass |
| Carbomer | 0.0005% by mass |
| Pentasodium pentetate | 0.0005% by mass |
| Phenoxyethanol | 0.0005% by mass |
| Methylparaben | 0.01% by mass |

(10) Milk-2

| | |
|---|---|
| Water | Remainder (about 70% by mass) |
| BG | 10% by mass |
| Dimethicone | 5% by mass |
| Mineral oil | 5% by mass |
| Glycerin | 5% by mass |
| Shea butter | 1% by mass |
| Polysorbate 60 | 1% by mass |
| Stearyl glycyrrhetinate | 1% by mass |
| Aqueous dispersion composition of Example 5 | 0.2% by mass |
| Aqueous dispersion composition of Example 2 | 0.2% by mass |
| Retinyl palmitate | 0.2% by mass |
| Scutellaria baicalensis root extract | 0.1% by mass |
| Cyclodextrin | 0.001% by mass |
| Turmeric root extract | 0.001% by mass |
| Prunus mume fruit extract | 0.0001% by mass |
| Eriobotrya japonica leaf extract | 0.0001% by mass |
| Laminaria ochroleuca extract | 0.0001% by mass |
| PEG-40 hydrogenated castor oil | 0.0001% by mass |
| Cetanol | 0.0001% by mass |

(continued)

| Sorbitan stearate | 0.0001% by mass |
|---|---|
| Corn oil | 0.0001% by mass |
| Caprylic/capric triglycerides | 0.0001% by mass |
| Lecithin | 0.0001% by mass |
| Hydrogenated lecithin | 0.0001% by mass |
| Polyglyceryl-10 oleate | 0.0001% by mass |
| Carbomer | 0.0001% by mass |
| Xanthan gum | 0.0001% by mass |
| Pentasodium pentetate | 0.0001% by mass |
| Sodium hydroxide | 0.0001% by mass |
| Phenoxyethanol | 0.0001% by mass |
| Methylparaben | 0.01% by mass |
| Ethylparaben | 0.01% by mass |

(11) UV Protect Milk

| Water | Remainder (about 40% by mass) |
|---|---|
| BG | 15% by mass |
| Ethylhexyl methoxycinnamate | 10% by mass |
| Cyclopentasiloxane | 10% by mass |
| Octocrylene | 10% by mass |
| Titanium dioxide | 10% by mass |
| Phenylbenzimidazole sulfonic acid | 1% by mass |
| Glyceryl stearate | 1% by mass |
| Glyceryl stearate (SE) | 1% by mass |
| Glycerin | 1% by mass |
| Stearyl glycyrrhetinate | 0.2% by mass |
| Aqueous dispersion composition of Example 5 | 0.1% by mass |
| Aqueous dispersion composition of Example 2 | 0.05% by mass |
| Retinyl palmitate | 0.05% by mass |
| Scutellaria baicalensis root extract | 0.05% by mass |
| Cyclodextrin | 0.01% by mass |
| Turmeric root extract | 0.001% by mass |
| Laminaria ochroleuca extract | 0.001% by mass |
| Cetanol | 0.001% by mass |
| Corn oil | 0.001% by mass |
| Caprylic/capric triglycerides | 0.001% by mass |
| Lecithin | 0.001% by mass |
| Polyglyceryl-10 oleate | 0.001% by mass |
| Xanthan gum | 0.001% by mass |
| Sodium polyacrylate | 0.001% by mass |
| Silica | 0.001% by mass |
| Alumina | 0.001% by mass |
| Sodium citrate | 0.001% by mass |
| Sodium hydroxide | 0.001% by mass |
| Phenoxyethanol | 0.01% by mass |
| Methylparaben | 0.01% by mass |

(12) Cream

| | |
|---|---|
| Water | Remainder (about 50% by mass) |
| Dimethicone | 20% by mass |
| Glycerin | 5% by mass |
| Vaseline | 5% by mass |
| Dipentaerythrityl hexa(hydroxystearate/stearate/rosinate) | 5% by mass |
| Glyceryl undecyl dimethicone | 5% by mass |
| Paraffin | 5% by mass |
| Stearyl glycyrrhetinate | 0.5% by mass |
| BG | 0.1% by mass |
| Aqueous dispersion composition of Example 5 | 0.02% by mass |
| Aqueous dispersion composition of Example 2 | 0.02% by mass |
| Retinyl palmitate | 0.005% by mass |
| Scutellaria baicalensis root extract | 0.005% by mass |
| Cyclodextrin | 0.00001% by mass |
| Turmeric root extract | 0.00001% by mass |
| Prunus mume fruit extract | 0.00001% by mass |
| Eriobotrya japonica leaf extract | 0.00001% by mass |
| Laminaria ochroleuca extract | 0.00001% by mass |
| Saccharomyces ferment lysate filtrate | 0.00001% by mass |
| PEG-9 polydimethylsiloxyethyl dimethicone | 0.00001% by mass |
| Cetyl dimethicone | 0.00001% by mass |
| Disteardimonium hectorite | 0.00001% by mass |
| Diphenylsiloxy phenyl trimethicone | 0.00001% by mass |
| Bisabolol | 0.00001% by mass |
| PEG-10 hydrogenated castor oil | 0.00001% by mass |
| Glyceryl caprylate | 0.00001% by mass |
| Corn oil | 0.00001% by mass |
| (Dimethicone/ phenyl vinyl dimethicone) crossploymer | 0.00001% by mass |
| (Dimethicone/ vinyl dimethicone) crossploymer | 0.00001% by mass |
| Caprylic/capric triglycerides | 0.00001% by mass |
| Lecithin | 0.00001% by mass |
| Polyglyceryl-10 oleate | 0.00001% by mass |
| Magnesium sulfate | 0.00001% by mass |
| Pentasodium pentetate | 0.00001% by mass |
| Phenoxyethanol | 0.01% by mass |

(13) Cosmetic Material for Face Pack

| | |
|---|---|
| Water | Remainder (about 78% by mass) |
| Butylene glycol | 5% by mass |
| Glycerin | 10% by mass |
| Glyceryl stearate | 0.3% by mass |
| Pentylene glycol | 1.0% by mass |
| Isotridecyl isononanoate | 1.0% by mass |
| Phenoxyethanol | 0.5% by mass |
| Carbomer | 0.3% by mass |
| Aqueous dispersion composition of Example 5 | 0.2% by mass |

(continued)

| | |
|---|---|
| Aqueous dispersion composition of Example 2 | 0.2% by mass |
| Retinyl palmitate | 0.1% by mass |
| Scutellaria baicalensis root extract | 0.05% by mass |
| Cyclodextrin | 0.001% by mass |
| Turmeric root extract | 0.001% by mass |
| Acetyl hydroxyproline | 0.1% by mass |
| Hydrolyzed collagen | 0.1% by mass |
| Ascorbic acid palmitate | 0.5% by mass |
| Magnesium ascorbyl phosphate | 0.2% by mass |
| Iodopropynyl butylcarbamate | 0.1% by mass |
| Ethylhexylglycerin | 0.1% by mass |
| Methylparaben | 0.05% by mass |

**Claims**

1. An aqueous dispersion composition comprising dispersed particles that include:

    a naturally occurring type Vitamin E compound comprising at least one selected from the group consisting of tocopherols and tocotrienols;
    retinyl palmitate;
    an oil agent having a calculated octanol-water partition coefficient (cLogPow) of from 0 to 12, wherein cLogPow is a value determined by a quantitative structure-activity correlation algorithm, or a value determined by a fragment method, and
    wherein the oil agent comprises tri(caprylic acid/capric acid)glyceride; and
    a phospholipid, wherein
    a particle diameter of the dispersed particles is from 20 nm to 90 nm,
    wherein the particle diameter is determined using the dynamic light scattering method as specified in the description.

2. The aqueous dispersion composition according to claim 1, wherein a content of the retinyl palmitate is from 0.001 times to 200 times a content of the naturally occurring type Vitamin E compound, in terms of mass ratio.

3. The aqueous dispersion composition according to claim 1 or 2,
    wherein the phospholipid is lecithin.

4. The aqueous dispersion composition according to any one of claim 1 to claim 3,
    wherein the cLogPow of the oil agent is from 2 to 12.

5. The aqueous dispersion composition according to claim 1, wherein the content of the naturally occurring type Vitamin E compound is from 0,0001% by mass to 10% by mass with respect to the total mass of the aqueous dispersion composition.

6. The aqueous dispersion composition according to claim 1, further comprising a surfactant.

7. The aqueous dispersion composition according to claim 1, wherein the content of the phospholipid is from 0.001% by mass to 10% by mass with respect to the total mass of the aqueous dispersion composition.

8. An external skin preparation comprising the aqueous dispersion composition according to any one of claim 1 to claim 7.

9. A functional food comprising the aqueous dispersion composition according to any one of claim 1 to claim 7.

**Patentansprüche**

1. Wässrige Dispersionszusammensetzung enthaltend dispergierte Partikel, die:

   eine natürlich vorkommende Vitamin E-Verbindung enthaltend mindestens eines, ausgewählt aus der Gruppe bestehend aus Tocopherolen und Tocotrienolen; Retinylpalmitat;
   ein Ölmittel, das einen berechneten Oktanol-Wasser-Verteilungskoeffizient (cLogPow) von 0 bis 12 hat, wobei cLogPow ein Wert ist, der durch einen quantitativen Struktur-Aktivitätskorrelationsalgorithmus bestimmt wird, oder ein Wert ist, der durch ein Fragmentverfahren bestimmt wird, und
   wobei das Ölmittel Tri(caprylsäure/caprinsäure)glycerid enthält; und
   ein Phospholipid
   enthalten, wobei
   ein Partikeldurchmesser der dispergierten Partikel von 20 nm bis 90 nm beträgt,
   wobei der Partikeldurchmesser unter Verwendung des in der Beschreibung spezifizierten dynamischen Lichtstreuungsverfahrens bestimmt wird.

2. Wässrige Dispersionszusammensetzung nach Anspruch 1, wobei ein Gehalt an dem Retinylpalmitat, bezogen auf das Masseverhältnis, das 0,001-fache bis 200-fache eines Gehalts an der natürlich vorkommenden Vitamin E-Verbindung beträgt.

3. Wässrige Dispersionszusammensetzung nach Anspruch 1 oder 2,
   wobei das Phospholipid Lecithin ist.

4. Wässrige Dispersionszusammensetzung nach einem der Ansprüche 1 bis 3,
   wobei der cLogPow des Ölmittels von 2 bis 12 beträgt.

5. Wässrige Dispersionszusammensetzung nach Anspruch 1, wobei der Gehalt an der natürlich vorkommenden Vitamin E-Verbindung von 0,0001 Masse% bis 10 Masse%, bezogen auf die Gesamtmasse der wässrigen Dispersionszusammensetzung, beträgt.

6. Wässrige Dispersionszusammensetzung nach Anspruch 1 ferner enthaltend ein Tensid.

7. Wässrige Dispersionszusammensetzung nach Anspruch 1, wobei der Gehalt an dem Phospholipid von 0,001 Masse% bis 10 Masse%, bezogen auf die Gesamtmasse der wässrigen Dispersionszusammensetzung, beträgt.

8. Externe Hautzubereitung enthaltend die wässrige Dispersionszusammensetzung nach einem der Ansprüche 1 bis 7.

9. Funktionales Nahrungsmittel enthaltend die wässrige Dispersionszusammensetzung nach einem der Ansprüche 1 bis 7.

**Revendications**

1. Composition de dispersion aqueuse, comprenant des particules dispersées incluant :

   un composé de vitamine E d'un type existant à l'état naturel comprenant au moins un élément sélectionné parmi le groupe consistant en des tocophérols et des tocotriénols ;
   du palmitate de rétinyle ;
   un agent huileux présentant un coefficient de partage octanol-eau calculé (cLogPow) allant de 0 à 12, dans laquelle cLogPow est une valeur déterminée par un algorithme de corrélation quantitatif structure-activité, ou une valeur déterminée par une méthode de fragment, et
   dans laquelle l'agent huileux comprend du tri(acide caprylique/acide caprique)glycéride, et
   un phospholipide, dans lequel
   un diamètre de particules des particules dispersées s'étend de 20 nm à 90 nm, et
   dans lequel le diamètre de particules est déterminé à l'aide d'une méthode de diffusion de la lumière dynamique comme spécifié dans la description.

2. Composition de dispersion aqueuse selon la revendication 1, dans laquelle une teneur en palmitate de rétinyle s'étend de 0,001 fois à 200 fois une teneur en composé de vitamine E d'un type existant à l'état naturel, en termes de rapport de masse.

3. Composition de dispersion aqueuse selon la revendication 1 ou 2, dans laquelle le phospholipide est de la lécithine.

4. Composition de dispersion aqueuse selon l'une quelconque des revendications 1 à 3, dans laquelle le cLogPow de l'agent huileux s'étend de 2 à 12.

5. Composition de dispersion aqueuse selon la revendication 1, dans laquelle la teneur en composé de vitamine E d'un type existant à l'état naturel s'étend de 0,0001 % en masse à 10 % en masse par rapport à la masse totale de la composition de dispersion aqueuse.

6. Composition de dispersion aqueuse selon la revendication 1, comprenant en outre un surfactant.

7. Composition de dispersion aqueuse selon la revendication 1, dans laquelle la teneur en phospholipide s'étend de 0,001 % en masse à 10 % en masse par rapport à la masse totale de la composition de dispersion aqueuse.

8. Préparation pour la peau à usage externe comprenant la composition de dispersion aqueuse selon l'une quelconque des revendications 1 à 7.

9. Aliment fonctionnel comprenant la composition de dispersion aqueuse selon l'une quelconque des revendications 1 à 7.

FIG.1

TOCOTRIENOL AMOUNT IN THE FOURTH
LAYER OF STRATUM CORNEUM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2133066 A
- JP 2001151664 A
- JP 2004168702 A
- JP 2004285078 A
- DE 102008035834 A1
- US 5376646 A
- KR 100614816 B1
- US 6066328 A
- JP 2004161733 A
- JP 2009256268 A